# EUROPEAN PATENT SPECIFICATION

(11) **EP 4 076 370 B1**
(45) Date of publication and mention of the grant of the patent: **29.07.2026**
(21) Application number: 20833978.8
(22) Date of filing: 08.12.2020
(51) Int. Cl.: A61Q 19/00, A61K 8/92, A61K 8/39, A61K 8/64, A61K 8/81

(54) **STABLE COMPOSITION COMPRISING COMBINATION OF SPECIFIC INGREDIENTS**
STABILE ZUSAMMENSETZUNG MIT EINER KOMBINATION SPEZIFISCHER INHALTSSTOFFE
COMPOSITION STABLE COMPRENANT UNE COMBINAISON D'INGRÉDIENTS SPÉCIFIQUES

(30) Priority: 18.12.2019 JP 2019228433
(43) Date of publication of application: 26.10.2022
(73) Proprietor: L'OREAL, 75008 Paris (FR); Mizuno, Tomoko, Kawasaki-shi, Kanagawa 213-0012 (JP); Maruyama, Kazuhiko, Kawasaki-shi, Kanagawa, 213-0012 (JP)
(72) Inventor: MIZUNO, Tomoko, Kawasaki-shi Kanagawa 2130012 (JP); MARUYAMA, Kazuhiko, Kawasaki-shi Kanagawa 2130012 (JP)
(74) Representative: Casalonga
(86) International application number: PCT/JP2020/046447
(87) International publication number: WO 2021/125118

(56) References cited:
- EP-A1- 3 391 872
- DATABASE Mintel GNPD [online] 1 September 2018 (2018-09-01), MINTEL: "Body Wash", XP055787833, Database accession no. 5988013
- DATABASE Mintel GNPD [online] 1 November 2015 (2015-11-01), MINTEL: "Night Cream Product Description", XP055787803, Database accession no. 3497019
- DATABASE Mintel GNPD [online] 1 June 2011 (2011-06-01), MINTEL: "Professional Shampoo Product Description", XP055787824, Database accession no. 1567648

## Description

### TECHNICAL FIELD

The present invention relates to a composition, preferably a cosmetic or dermatological composition, which comprises a combination of specific ingredients.

### BACKGROUND ART

In recent years, the milky lotion market has been evolving in north Asia, and expanding worldwide. In such a field, oil-in-water emulsions are known from EP 3 391 872 and comprise a first and a second surfactant. Said first surfactant forms an L_{α} phase in at least one aqueous solution having a concentration of 40 to 80% by mass, and said second surfactant forms an H₁ phase in at least one aqueous solution having a concentration of 40 to 80% by mass.

There are not many varieties of milky lotions in terms of viscosity, because they tend to have an extremely low viscosity.

However, the lower the viscosity becomes, the less stable the milky lotion becomes. Thus, the color, odor and pH of the milky lotion may change. Also, phase separation or creaming due to agglomeration of oil(s) in the milky lotion may occur.

Furthermore, there has been a need for milky lotions which can provide good texture such as smooth skin finish.

### DISCLOSURE OF INVENTION

An objective of the present invention is to provide a composition which can achieve a low viscosity but is stable, and can provide good texture such as a smooth feeling to the touch after application.

The above objective of the present invention can be achieved by a composition comprising:
(a) at least one oil;
(b) at least one polyglyceryl fatty acid ester having an HLB of 8 to 11;
(c) at least one anionic surfactant;
(d) at least one acrylic thickener; and
(e) water,
wherein
the specific gravity of the (a) oil(s) at 25°C is more than 0.96 and preferably more than 0.97.and
the (c) anionic surfactant is selected from the compounds resented by the formula (A): 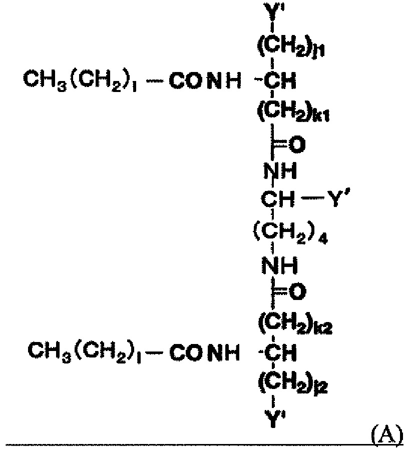 wherein
   Y' independently denotes a carboxylic acid group or an alkaline salt of a carboxylic acid group such as a sodium salt of a carboxylic acid group,
   j1, k1, j2 and k2 denote integers such that (j1, k1, j2, k2) = any of (2,0,2,0), (2,0,0,2), (0,2,2,0) and (0,2,0,2), and
   1 denotes an integer from 6 to 16.

The (a) oil may be selected from triglyceride oils.

The (a) oil(s) in the composition according to the present invention may range from 0.01% to 25% by weight, preferably from 0.1% to 20% by weight, and more preferably from 1% to 15% by weight, relative to the total weight of the composition.

The fatty acid moiety of the (b) polyglyceryl fatty acid ester may comprise 12 to 24 carbon atoms, preferably 14 to 22 carbon atoms, and more preferably 16 to 20 carbon atoms.

The (b) polyglyceryl fatty acid ester may comprise 4 to 10 glycerol units, preferably 4 to 9 glycerol units, and more preferably 4 to 8 glycerol units.

The (b) polyglyceryl fatty acid ester(s) in the composition according to the present invention may range from 0.01% to 10% by weight, preferably from 0.05% to 5% by weight, and more preferably from 0.1% to 2% by weight, relative to the total weight of the composition.

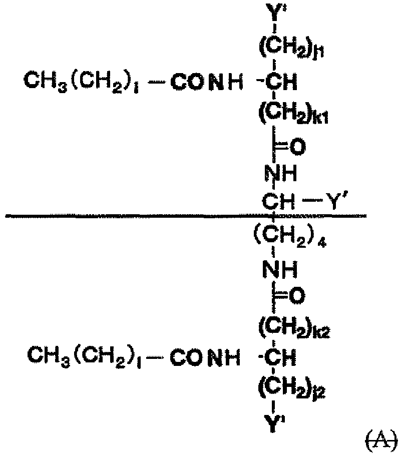

The (c) anionic surfactant may be selected from the group consisting of sodium dilauramidoglutamide lysine, sodium dimyristoylglutamide lysine, and sodium distearoylglutamide lysine, and a mixture thereof.

The amount of the (c) anionic surfactant(s) in the composition according to the present invention may range from 0.01% to 10% by weight, preferably from 0.03% to 5% by weight, and more preferably from 0.05% to 0.5% by weight, relative to the total weight of the composition.

The (d) acrylic thickener may be selected from polyacrylate crosspolymer-6, acrylates/C10-30 alkyl acrylate crosspolymer and a mixture thereof.

The amount of the (d) acrylic thickener(s) in the composition according to the present invention may range from 0.01% to 5% by weight, preferably from 0.05% to 3% by weight, and more preferably from 0.1% to 1% by weight, relative to the total weight of the composition.

The amount of the (e) water in the composition according to the present invention may range from 50% to 90% by weight, preferably from 60% to 85% by weight, and more preferably from 70% to 80% by weight, relative to the total weight of the composition.

The composition according to the present invention may be in the form of an O/W emulsion.

The composition according to the present invention may be a cosmetic composition, preferably a cosmetic composition for a keratin substance, and more preferably a cosmetic composition for skin.

The present invention also relates to a cosmetic process for treating a keratin substance, comprising the step of applying the composition according to the present invention to the keratin substance.

### BEST MODE FOR CARRYING OUT THE INVENTION

After diligent research, the inventors have discovered that it is possible to provide a composition which can achieve a low viscosity but is stable, and can provide good texture such as a smooth feeling to the touch after application.

The composition according to the present invention may be characterized by a combination of:
at least one relatively heavy oil;
at least one second polyglyceryl fatty acid ester having a specific HLB value range;
at least one anionic surfactant; and
at least one acrylic thickener.

Thus, one aspect of the present invention is a composition comprising:
(a) at least one oil;
(b) at least one polyglyceryl fatty acid ester having an HLB of 8 to 11;
(c) at least one anionic surfactant as defined in the present invention;
(d) at least one acrylic thickener; and
(e) water,
wherein
the specific gravity of the (a) oil(s) at 25°C is more than 0.96 and preferably more than 0.97.

The term "specific gravity" is well known as a ratio of the density of a target substance to the density of a standard substance. The "specific gravity" here means a ratio of the density of oil(s) to the density of water (1 g/cm³).

The composition according to the present invention can achieve a low viscosity but is stable. Therefore, even if the composition according to the present invention has a low viscosity, phase separation or creaming of the composition can be prevented or reduced.

The term "stable" here reflects temperature stability and means that the aspect of a composition does not change for a long period of time at a variety of constant temperatures such as 4°C, 25°C, 40°C and 45°C.

Also, the composition according to the present invention can provide good texture such as a smooth feeling to the touch after application. In other words, the composition according to the present invention is not sticky after being applied onto a keratin substance such as skin. The term "sticky" here means a property which provides a tacky feeling to the skin.

Thus, the composition according to the present invention is, in particular, suitable for a milky lotion with a low viscosity.

Hereinafter, the composition according to the present invention will be explained in a more detailed manner.

### [Oil]

The composition according to the present invention comprises (a) at least one oil. If two or more oils are used, they may be the same or different.

Here, "oil" means a fatty compound or substance which is in the form of a liquid or a paste (non-solid) at room temperature (25°C) under atmospheric pressure (760 mmHg). As the oils, those generally used in cosmetics can be used alone or in combination thereof. These oils may be volatile or non-volatile.

The (a) oil may be a non-polar oil such as a hydrocarbon oil, a silicone oil, or the like; a polar oil such as a plant or animal oil and an ester oil or an ether oil; or a mixture thereof.

The (a) oil may be selected from the group consisting of oils of plant or animal origin, synthetic oils, silicone oils, hydrocarbon oils, and fatty alcohols.

As examples of plant oils, mention may be made of, for example, linseed oil, camellia oil, macadamia nut oil, corn oil, mink oil, olive oil, avocado oil, sasanqua oil, castor oil, safflower oil, jojoba oil, sunflower oil, almond oil, rapeseed oil, sesame oil, soybean oil, peanut oil, and mixtures thereof.

As examples of animal oils, mention may be made of, for example, squalene and squalane.

As examples of synthetic oils, mention may be made of alkane oils such as isododecane and isohexadecane, ester oils, ether oils, and artificial triglycerides.

The ester oils are preferably liquid esters of saturated or unsaturated, linear or branched C₁-C₂₆ aliphatic monoacids or polyacids and of saturated or unsaturated, linear or branched C₁-C₂₆ aliphatic monoalcohols or polyalcohols, the total number of carbon atoms of the esters being greater than or equal to 10.

Preferably, for the esters of monoalcohols, at least one from among the alcohol and the acid from which the esters of the present invention are derived is branched.

Among the monoesters of monoacids and of monoalcohols, mention may be made of ethyl palmitate, ethyl hexyl palmitate, isopropyl palmitate, dicaprylyl carbonate, alkyl myristates such as isopropyl myristate or ethyl myristate, isocetyl stearate, 2-ethylhexyl isononanoate, isononyl isononanoate, isodecyl neopentanoate, and isostearyl neopentanoate.

Esters of C₄-C₂₂ dicarboxylic or tricarboxylic acids and of C₁-C₂₂ alcohols, and esters of monocarboxylic, dicarboxylic, or tricarboxylic acids and of non-sugar C₄-C₂₆ dihydroxy, trihydroxy, tetrahydroxy, or pentahydroxy alcohols may also be used.

Mention may especially be made of: diethyl sebacate; isopropyl lauroyl sarcosinate; diisopropyl sebacate; bis(2-ethylhexyl) sebacate; diisopropyl adipate; di-n-propyl adipate; dioctyl adipate; bis(2-ethylhexyl) adipate; diisostearyl adipate; bis(2-ethylhexyl) maleate; triisopropyl citrate; triisocetyl citrate; triisostearyl citrate; glyceryl trilactate; glyceryl trioctanoate; trioctyldodecyl citrate; trioleyl citrate; neopentyl glycol diheptanoate; diethylene glycol diisononanoate.

As ester oils, one can use sugar esters and diesters of C₆-C₃₀ and preferably C₁₂-C₂₂ fatty acids.

It is recalled that the term "sugar" means oxygen-bearing hydrocarbon-based compounds containing several alcohol functions, with or without aldehyde or ketone functions, and which comprise at least 4 carbon atoms. These sugars may be monosaccharides, oligosaccharides, or polysaccharides.

Examples of suitable sugars that may be mentioned include sucrose (or saccharose), glucose, galactose, ribose, fucose, maltose, fructose, mannose, arabinose, xylose, and lactose, and derivatives thereof, especially alkyl derivatives, such as methyl derivatives, for instance methylglucose.

The sugar esters of fatty acids may be chosen especially from the group comprising the esters or mixtures of esters of sugars described previously and of linear or branched, saturated or unsaturated C₆-C₃₀ and preferably C₁₂-C₂₂ fatty acids. If they are unsaturated, these compounds may have one to three conjugated or non-conjugated carbon-carbon double bonds.

The esters according to this variant may also be selected from monoesters, diesters, triesters, tetraesters, and polyesters, and mixtures thereof.

These esters may be, for example, oleates, laurates, palmitates, myristates, behenates, cocoates, stearates, linoleates, linolenates, caprates, and arachidonates, or mixtures thereof such as, especially, oleopalmitate, oleostearate, and palmitostearate mixed esters, as well as pentaerythrityl tetraethyl hexanoate.

More particularly, use is made of monoesters and diesters and especially sucrose, glucose, or methylglucose monooleates or dioleates, stearates, behenates, oleopalmitates, linoleates, linolenates, and oleostearates.

An example that may be mentioned is the product sold under the name Glucate^{®} DO by the company Amerchol, which is a methylglucose dioleate.

As examples of preferable ester oils, mention may be made of, for example, diisopropyl adipate, dioctyl adipate, 2-ethylhexyl hexanoate, ethyl laurate, cetyl octanoate, octyldodecyl octanoate, isodecyl neopentanoate, myristyl propionate, 2-ethylhexyl 2-ethylhexanoate, 2-ethylhexyl octanoate, 2-ethylhexyl caprylate/caprate, methyl palmitate, ethyl palmitate, isopropyl palmitate, dicaprylyl carbonate, isopropyl lauroyl sarcosinate, isononyl isononanoate, ethylhexyl palmitate, isohexyl laurate, hexyl laurate, isocetyl stearate, isopropyl isostearate, isopropyl myristate, isodecyl oleate, glyceryl tri(2-ethylhexanoate), pentaerythrithyl tetra(2-ethylhexanoate), 2-ethylhexyl succinate, diethyl sebacate, and mixtures thereof.

As examples of artificial triglycerides, mention may be made of, for example, capryl caprylyl glycerides, glyceryl trimyristate, glyceryl tripalmitate, glyceryl trilinolenate, glyceryl trilaurate, glyceryl tricaprate, glyceryl tricaprylate, glyceryl tri(caprate/caprylate), glyceryl tri(caprate/caprylate/linolenate) and glyceryl tri(caprate/caprylate/succinate).

As examples of silicone oils, mention may be made of, for example, linear organopolysiloxanes such as dimethylpolysiloxane, methylphenylpolysiloxane, methylhydrogenpolysiloxane, and the like; cyclic organopolysiloxanes such as cyclohexasiloxane, octamethylcyclotetrasiloxane, decamethylcyclopentasiloxane, dodecamethylcyclohexasiloxane, and the like; and mixtures thereof.

Preferably, the silicone oil is chosen from liquid polydialkylsiloxanes, especially liquid polydimethylsiloxanes (PDMS) and liquid polyorganosiloxanes comprising at least one aryl group.

These silicone oils may also be organomodified. The organomodified silicones that can be used in accordance with the present invention are silicone oils as defined above and comprise in their structure one or more organofunctional groups attached via a hydrocarbon-based group.

Organopolysiloxanes are defined in greater detail in Walter Noll's Chemistry and Technology of Silicones (1968), Academic Press. They may be volatile or non-volatile.

When they are volatile, the silicones are more particularly chosen from those having a boiling point of between 60°C and 260°C, and even more particularly from:
(i) cyclic polydialkylsiloxanes comprising from 3 to 7 and preferably 4 to 5 silicon atoms. These are, for example, octamethylcyclotetrasiloxane sold in particular under the name Volatile Silicone^{®} 7207 by Union Carbide or Silbione^{®} 70045 V2 by Rhodia, decamethylcyclopentasiloxane sold under the name Volatile Silicone^{®} 7158 by Union Carbide, Silbione^{®} 70045 V5 by Rhodia, and dodecamethylcyclopentasiloxane sold under the name Silsoft 1217 by Momentive Performance Materials, and mixtures thereof. Mention may also be made of cyclocopolymers of the type such as dimethylsiloxane/methylalkylsiloxane, such as Silicone Volatile^{®} FZ 3109 sold by the company Union Carbide, of the formula: 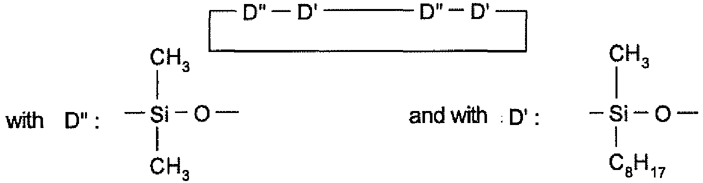 Mention may also be made of mixtures of cyclic polydialkylsiloxanes with organosilicon compounds, such as the mixture of octamethylcyclotetrasiloxane and tetratrimethylsilylpentaerythritol (50/50) and the mixture of octamethylcyclotetrasiloxane and oxy-1,1'-bis(2,2,2',2',3,3'-hexatrimethylsilyloxy)neopentane; and
(ii) linear volatile polydialkylsiloxanes containing 2 to 9 silicon atoms and having a viscosity of less than or equal to 5×10⁻⁶ m²/s at 25°C. An example is decamethyltetrasiloxane sold in particular under the name SH 200 by the company Toray Silicone. Silicones belonging to this category are also described in the article published in Cosmetics and Toiletries, Vol. 91, Jan. 76, pp. 27-32, Todd & Byers, Volatile Silicone Fluids for Cosmetics*.* The viscosity of the silicones is measured at 25°C according to ASTM standard 445 Appendix C.

Non-volatile polydialkylsiloxanes may also be used. These non-volatile silicones are more particularly chosen from polydialkylsiloxanes, among which mention may be made mainly of polydimethylsiloxanes containing trimethylsilyl end groups.

Among these polydialkylsiloxanes, mention may be made, in a non-limiting manner, of the following commercial products:
- the Silbione^{®} oils of the 47 and 70 047 series or the Mirasil^{®} oils sold by Rhodia, for instance the oil 70 047 V 500 000;
- the oils of the Mirasil^{®} series sold by the company Rhodia;
- the oils of the 200 series from the company Dow Coming, such as DC200 with a viscosity of 60 000 mm²/s; and
- the Viscasil^{®} oils from General Electric and certain oils of the SF series (SF 96, SF 18) from General Electric.

Mention may also be made of polydimethylsiloxanes containing dimethylsilanol end groups known under the name dimethiconol (CTFA), such as the oils of the 48 series from the company Rhodia.

Among the silicones containing aryl groups, mention may be made of polydiarylsiloxanes, especially polydiphenylsiloxanes and polyalkylarylsiloxanes such as phenyl silicone oil.

The phenyl silicone oil may be chosen from the phenyl silicones of the following formula: 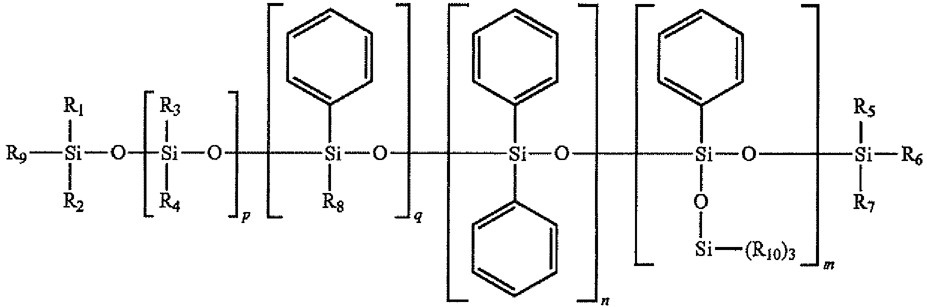 in which
R₁ to R₁₀, independently of each other, are saturated or unsaturated, linear, cyclic or branched C₁-C₃₀ hydrocarbon-based radicals, preferably C₁-C₁₂ hydrocarbon-based radicals, and more preferably C₁-C₆ hydrocarbon-based radicals, in particular methyl, ethyl, propyl, or butyl radicals, and
m, n, p, and q are, independently of each other, integers of 0 to 900 inclusive, preferably 0 to 500 inclusive, and more preferably 0 to 100 inclusive,
with the proviso that the sum n+m+q is other than 0.

Examples that may be mentioned include the products sold under the following names:
- the Silbione^{®} oils of the 70 641 series from Rhodia;
- the oils of the Rhodorsil^{®} 70 633 and 763 series from Rhodia;
- the oil Dow Coming 556 Cosmetic Grade Fluid from Dow Corning;
- the silicones of the PK series from Bayer, such as the product PK20;
- certain oils of the SF series from General Electric, such as SF 1023, SF 1154, SF 1250, and SF 1265.

As the phenyl silicone oil, phenyl trimethicone (R₁ to R₁₀ are methyl; p, q, and n = 0; m=1 in the above formula) is preferable.

The organomodified liquid silicones may especially contain polyethyleneoxy and/or polypropyleneoxy groups. Mention may thus be made of the silicone KF-6017 proposed by Shin-Etsu, and the oils Silwet^{®} L722 and L77 from the company Union Carbide.

Hydrocarbon oils may be chosen from:
- linear or branched, optionally cyclic, C₆-C₁₆ lower alkanes. Examples that may be mentioned include hexane, undecane, dodecane, tridecane, and isoparaffins, for instance isohexadecane, isododecane, and isodecane; and
- linear or branched hydrocarbons containing more than 16 carbon atoms, such as liquid paraffins, liquid petroleum jelly, polydecenes and hydrogenated polyisobutenes such as Parleam^{®}, and squalane.

It may be preferable that the (a) oil comprises no silicone oil.

As preferable examples of hydrocarbon oils, mention may be made of, for example, linear or branched hydrocarbons such as isohexadecane, isododecane, squalane, mineral oil (e.g., liquid paraffin), paraffin, vaseline or petrolatum, naphthalenes, and the like; hydrogenated polyisobutene, isoeicosan, and decene/butene copolymer; and mixtures thereof.

The term "fatty" in the fatty alcohol means the inclusion of a relatively large number of carbon atoms. Thus, alcohols which have 4 or more, preferably 6 or more, and more preferably 12 or more carbon atoms are encompassed within the scope of fatty alcohols. The fatty alcohol may be saturated or unsaturated. The fatty alcohol may be linear or branched.

The fatty alcohol may have the structure R-OH wherein R is chosen from saturated and unsaturated, linear and branched radicals containing from 4 to 40 carbon atoms, preferably from 6 to 30 carbon atoms, and more preferably from 12 to 20 carbon atoms. In at least one embodiment, R may be chosen from C₁₂-C₂₀ alkyl and C₁₂-C₂₀ alkenyl groups. R may or may not be substituted with at least one hydroxyl group.

As examples of the fatty alcohol, mention may be made of lauryl alcohol, cetyl alcohol, stearyl alcohol, isostearyl alcohol, behenyl alcohol, undecylenyl alcohol, myristyl alcohol, octyldodecanol, hexyldecanol, oleyl alcohol, linoleyl alcohol, palmitoleyl alcohol, arachidonyl alcohol, erucyl alcohol, and mixtures thereof.

It is preferable that the fatty alcohol be a saturated fatty alcohol.

Thus, the fatty alcohol may be selected from straight or branched, saturated or unsaturated C₆-C₃₀ alcohols, preferably straight or branched, saturated C₆-C₃₀ alcohols, and more preferably straight or branched, saturated C₁₂-C₂₀ alcohols.

The term "saturated fatty alcohol" here means an alcohol having a long aliphatic saturated carbon chain. It is preferable that the saturated fatty alcohol be selected from any linear or branched, saturated C₆-C₃₀ fatty alcohols. Among the linear or branched, saturated C₆-C₃₀ fatty alcohols, linear or branched, saturated C₁₂-C₂₀ fatty alcohols may preferably be used. Any linear or branched, saturated C₁₆-C₂₀ fatty alcohols may more preferably be used. Branched C₁₆-C₂₀ fatty alcohols may even more preferably be used.

As examples of saturated fatty alcohols, mention may be made of lauryl alcohol, cetyl alcohol, stearyl alcohol, isostearyl alcohol, behenyl alcohol, undecylenyl alcohol, myristyl alcohol, octyldodecanol, hexyldecanol, and mixtures thereof. In one embodiment, cetyl alcohol, stearyl alcohol, octyldodecanol, hexyldecanol, or a mixture thereof (e.g., cetearyl alcohol) as well as behenyl alcohol, can be used as a saturated fatty alcohol.

According to at least one embodiment, the fatty alcohol used in the composition according to the present invention is preferably chosen from cetyl alcohol, octyldodecanol, hexyldecanol, and mixtures thereof.

The (a) oil may be chosen from oils with a molecular weight below 600 g/mol.

Preferably, the (a) oil has a low molecular weight such as below 600 g/mol, chosen among ester oils with a short hydrocarbon chain or chains (C₁-C₁₂) (e.g., isopropyl lauroyl sarcosinate, isopropyl myristate, isopropyl palmitate, isononyl isononanoate, and ethyl hexyl palmitate), silicone oils (e.g., volatile silicones such as cyclohexasiloxane), hydrocarbon oils (e.g., isododecane, isohexadecane, and squalane), branched and/or unsaturated fatty alcohol (C₁₂-C₃₀) type oils such as octyldodecanol and oleyl alcohol, and ether oils such as dicaprylylether.

It is preferable that the (a) oil be chosen from triglyceride oils.

The triglyceride oil comprises at least one triglyceride. The triglyceride may be referred to as a triacyl glycerol, and three fatty acids or two fatty acids and one non-fatty acid, and one glycerol are esterified in the triglyceride.

The fatty acid may have, for example, 4 or more, 6 or more, 8 or more, or 10 or more carbon atoms, and 30 or less, 28 or less, 26 or less, or 24 or less carbon atoms. The fatty acid may have a different carbon chain length of, for example, from 4 to 30 carbon atoms, preferably from 6 to 28 carbon atoms, more preferably from 8 to 26 carbon atoms, and even more preferably from 10 to 24 carbon atoms. The carbon chain may be linear or branched.

The fatty acid may be saturated or unsaturated.

As examples of the saturated fatty acid, mention may be made of, for example, palmitic acid, heptadecanoic acid, stearic acid, nonadecanoic acid, arachidic acid, behenic acid, tetradocosanoic acid, hexadocosanoic acid, and octadocosanoic acid.

As examples of the unsaturated fatty acid, mention may be made of, for example, palmitoleic acid, oleic acid, elaidic acid, vaccenic acid, gadoleic acid, eicosenoic acid, erucic acid, nervonic acid, linoleic acid, eicosadienoic acid, docosadienoic acid, linolenic acid, pinolenic acid, eleostearic acid, mead acid, eicosatrienoic acid, stearidonic acid, arachidonic acid, eicosatetraenoic acid, adrenic acid, bosseopentaenoic acid, eicosapentaenoic acid, osbond acid, clupanodonic acid, tetracosapentaenoic acid, docosahexaenoic acid, and nisinic acid.

Examples of non-fatty acid may be dicarboxylic acids which may have, for example, 1 or more, 2 or more, 3 or more, or 4 or more carbon atoms, and 12 or less, 10 or less, 8 or less, or 6 or less carbon atoms. The non-fatty acid may have a different carbon chain length of, for example, from 1 to 12 carbon atoms, preferably from 2 to 10 carbon atoms, more preferably from 3 to 8 carbon atoms, and even more preferably from 4 to 6 carbon atoms. The carbon chain may be linear or branched.

The non-fatty acid, preferably dicarboxylic acid, may be saturated or unsaturated.

As examples of the saturated non-fatty acid, preferably saturated dicarboxylic acid, mention may be made of, for example, oxalic acid, malonic acid, succinic acid, glutaric acid, adipic acid, pimelic acid, suberic acid, azelaic acid and sebacic acid.

As examples of the unsaturated fatty acid, mention may be made of, for example, maleic acid, fumaric acid, citraconic acid, mesaconic acid and 2-pentenoic acid.

The triglyceride oil which may be suitable for the present invention is of plant origin. In other words, it is preferable that the triglyceride oil is selected from plant oils.

The plant oil may be selected from plant-extracted oils, plant-extracted butters, and mixtures thereof.

Among the plant-extracted oils, the following may be cited: jojoba oil, babassu oil, sunflower oil, olive oil, canola oil, coconut oil, meadowfoam seed oil; Brazil nut oil, marula oil, maize oil, argan oil, soybean oil, marrow oil, grapeseed oil, linseed oil, sesame oil, hazelnut oil, apricot oil, macadamia oil, arara oil, coriander oil, castor oil, avocado oil, shea butter oil, rapeseed oil, and copra oil.

Among the plant-extracted butters, the following may be cited: shea butter, Nilotica shea butter (Butyrospermum parkii), galam butter (Butyrospermum parkii), Borneo butter or fat or tengkawang tallow (Shorea stenoptera), shorea butter, illipe butter, madhuca butter or (Bassia) Madhuca longifolia butter, mowrah butter (Madhuca latifolia), katiau butter (Madhuca mottleyana), phulwara butter (M. butyracea), mango butter (Mangifera indica), murumuru butter (Astrocaryum murumuru), kokum butter (Garcinia indica), ucuuba butter (Virola sebifera), tucuma butter, painya (kpangnan) butter (Pentadesma butyracea), coffee butter (Coffea arabica), apricot butter (Prunus armeniaca), macadamia butter (Macadamia ternifolia), grapeseed butter (Vitis vinifera), avocado butter (Persea gratissima), olive butter (Olea europaea), sweet almond butter (Prunus amygdalus dulcis), cocoa butter (Theobroma cacao), and sunflower butter.

It is more preferable that the triglycride oil be selected from caprylic/capric/succinic triglyceride, shear butter, and a mixture thereof.

It is preferable that the amount of the triglyceride oil(s) in the (a) oil(s) in the composition according to the present invention be 50% by weight or more, preferably 60% by weight or more, and more preferably 70% by weight or more, relative to the total weight of the (a) oil(s).

According to the present invention, the specific gravity of the (a) oil(s) at 25°C is preferably more than 0.96 and mere-preferably more than 0.97.

In usual, oils have a lower specific gravity as compared to water. If the viscosity of a composition including oil and water is low, the oil can relatively easily float up in water which may cause agglomeration of oil droplets and phase separation between oil and water.

However, as the (a) oil used in the present invention has a higher specific gravity, even if the composition has a low viscosity, floating up of oil droplets in water is not easy so that agglomeration of oil droplets and phase separation between oil and water can be prevented or reduced.

If two or more (a) oils are used, the specific gravity can be determined by the average of the specific gravities of all the (a) oils.

The amount of the (a) oil(s) in the composition according to the present invention may be 0.01 % by weight or more, preferably 0.1% by weight or more, and more preferably 1% by weight or more, relative to the total weight of the composition.

The amount of the (a) oil(s) in the composition according to the present invention may be 25% by weight or less, preferably 20% by weight or less, and more preferably 15% by weight or less, relative to the total weight of the composition.

The amount of the (a) oil(s) in the composition according to the present invention may be from 0.01% to 25% by weight, preferably from 0.1% to 20% by weight, and more preferably from 1% to 15% by weight, relative to the total weight of the composition.

The (a) oil(s) can form a fatty phase of the composition according to the present invention.

If the composition according to the present invention is in the form of an O/W emulsion, the (a) oil in the composition according to the present invention can form dispersed fatty phases in the O/W emulsion.

### [Polyglyceryl Fatty Acid Ester]

The composition according to the present invention comprises (b) at least one polyglyceryl fatty acid ester having an HLB value of 8 to 11. A single type of (b) polyglyceryl fatty acid ester may be used, but two or more different types of (b) polyglyceryl fatty acid ester may be used in combination.

The (b) polyglyceryl fatty acid ester can function as a surfactant, in particular a nonionic surfactant.

The (b) polyglyceryl fatty acid may have an HLB value of 8.0 to 11.0, preferably 8.5 to 11.0, more preferably 9.0 to 11.0, and even more preferably 9.5 to 11.0. If the HLB value of the (b) polyglyceryl fatty acid ester is less than 8.0, the stability of the composition according to the present invention may be deteriorated.

If two or more (b) polyglyceryl fatty acid esters are used, the HLB value is determined by the weighted average of the HLB values of all the (b) polyglyceryl fatty acid esters.

The (b) polyglyceryl fatty acid ester may be chosen from mono, di, tri and more esters of saturated or unsaturated fatty acid(s).

The fatty acid for the fatty acid moiety of the (b) polyglyceryl fatty acid ester or the fatty acid moiety of the (b) polyglyceryl fatty acid ester may include 12 to 24 carbon atoms, preferably 14 to 22 carbon atoms, and more preferably 16 to 20 carbon atoms. If the (b) polyglyceryl fatty acid ester has two or more fatty acid moieties, the fatty acid for each of the fatty acid moiety of the (b) polyglyceryl fatty acid ester or each of the fatty acid moiety of the (b) polyglyceryl fatty acid ester may include 12 to 24 carbon atoms, preferably 14 to 22 carbon atoms, and more preferably 16 to 20 carbon atoms.

The fatty acid for the fatty acid moiety of the (b) polyglyceryl fatty acid ester may be saturated or unsaturated, and may be selected from lauric acid, myristic acid, stearic acid, isostearic acid and oleic acid, linoleic acid and behenic acid

It is preferable that the (b) polyglyceryl fatty acid ester comprise 4 to 10 glycerol units, preferably 4 to 9 glycerol units, and more preferably 4 to 8 glycerol units.

The (b) polyglyceryl fatty acid ester(s) may be selected from the group consisting of PG2 sesquicaprylate (HLB: about 8), PG2 caprate (HLB: 9.5), PG2 laurate (HLB: 8.5), PG4 oleate (HLB: 8.8), PG4 laurate (HLB: 10.3), PG4 isostearate (HLB: 8.2), PG5 laurate (HLB: 10.5), PG6 dioleate (HLB: 9.8), PG6 isostearate (HLB: 10.8), PG6 distearate (HLB: 8), PG10 trilaurate (HLB: 10.4), PG10 distearate (HLB: about 9), PG10 tristearate (HLB: 8), PG10 diisostearate (HLB: 10), PG10 triisostearate (HLB: 8), PG10 dioleate (HLB: about 11), PG10 tricocoate (HLB: 9), and mixtures thereof.

The amount of the (b) polyglyceryl fatty acid ester(s) in the composition according to the present invention may be 0.01% by weight or more, preferably 0.05% by weight or more, and more preferably 0.1% by weight or more, relative to the total weight of the composition.

On the other hand, the amount of the (b) polyglyceryl fatty acid ester(s) in the composition according to the present invention may be 10% by weight or less, preferably 5% by weight or less, and more preferably 2% by weight or less, relative to the total weight of the composition.

The amount of the (b) polyglyceryl fatty acid ester(s) in the composition according to the present invention may range from 0.01% to 10% by weight, preferably from 0.05% to 5% by weight, more preferably from 0.1% to 2% by weight, relative to the total weight of the composition.

The weight ratio of the amount of the (b) polyglyceryl fatty acid ester(s)/the amount of the (a) oil(s) in the composition according to the present invention may range from 0.001 to 0.3, preferably from 0.005 to 0.2, and more preferably from 0.01 to 0.1.

### [Anionic Surfactant]

The composition according to the present invention comprises (c) at least one anionic surfactant. A single type of anionic surfactant may be used, but two or more different types of anionic surfactant may be used in combination.

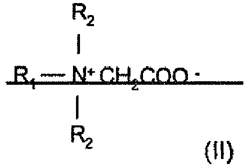

The (c) anionic surfactant is selected from the compounds represented by the formula (A): 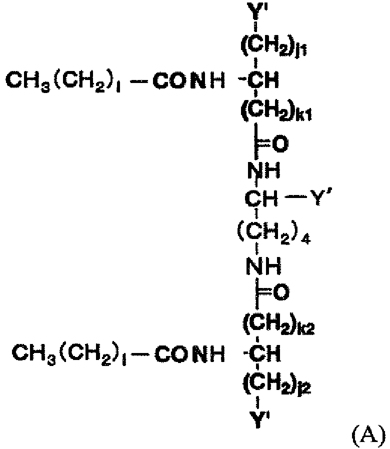 wherein
Y' independently denotes a carboxylic acid group or an alkaline salt of a carboxylic acid group such as a sodium salt of a carboxylic acid group,
j1, k1, j2 and k2 denote an integer such that (j1, k1, j2, k2) = any of (2,0,2,0), (2,0,0,2), (0,2,2,0) and (0,2,0,2), and
l denotes an integer from 6 to 16.

Preferably, in formula (A), L denotes an integer from 8 to 12, j1 = j2 = 0, and k1=k2 = 2.

Most preferably, in formula (A), Y' is -COONa , j1 = j2 = 0, k1=k2 = 2 ; and L = 10.

As the above compound represented by formula (A), mention may be made of sodium dilauramidoglutamide lysine, sodium dimyristoylglutamide lysine, and sodium distearoylglutamide lysine. Sodium dilauramidoglutamide lysine is in particular preferable. Sodium dilauramidoglutamide lysine is marketed as PELLICER L-30 by Asahi Chemicals as an aqueous solution at a concentration of 29% by weight relative to the total weight of the aqueous solution or PELLICER LB-30G by Asahi Chemicals as an aqueous solution at a concentration of 27.3% by weight relative to the total weight of the aqueous solution.

The compound represented by formula (A) can be prepared by, for example, reacting a long chain N-acyl acidic amino acid anhydride with a basic amino acid, such as lysine, in water and/or a mixed solvent of water and organic solvent(s), or in inert organic solvent(s) such as tetrahydrofuran, benzene, toluene, xylene tetrachloromethane, chloroform, acetone or the like, or without any solvent, at -5°C to 200°C, preferably 5°C to 100°C, and more preferably 0°C to 60°C.

The amount of the (c) anionic surfactant(s) in the composition according to the present invention may be 0.01% by weight or more, preferably 0.03% by weight or more, and more preferably 0.05% by weight or more relative to the total weight of the composition.

On the other hand, the amount of the (c) anionic surfactant(s) in the composition according to the present invention may be 10% by weight or less, preferably 5% by weight or less, and more preferably 0.5% by weight or less relative to the total weight of the composition.

Thus, the amount of the (c) anionic surfactant(s) in the composition according to the present invention may range from 0.01% to 10% by weight, preferably from 0.03% to 5% by weight, and more preferably from 0.05% to 0.5% by weight relative to the total weight of the composition.

The weight ratio of the amount of the (c) anionic surfactant(s)/the amount of the (a) oil(s) in the composition according to the present invention may range from 0.001 to 0.3, preferably from 0.003 to 0.2, and more preferably from 0.005 to 0.1.

### [Acrylic Thickener]

The composition according to the present invention comprises (d) at least one acrylic thickener. A single type of acrylic thickener may be used, but two or more different types of acrylic thickener may be used in combination.

The term "acrylic thickener" as used herein refers to polymers based upon one or more (meth)acrylic acid (and corresponding (meth)acrylate) monomers or similar monomers.

According to preferred embodiments, the acrylic thickener is an anionic acrylic polymer comprising at least one monomer performing a weak acid function such as, for example, acrylic acid, methacrylic acid, itaconic acid, crotonic acid, maleic acid and/or fumaric acid.

According to preferred embodiments, the acrylic thickener is an anionic acrylic polymer further comprising at least one monomer performing a strong acid function such as, for example, monomers having a function of the sulfonic acid type or phosphonic acid type, such as 2-acrylamido-2-methylpropane sulfonic acid (AMPS).

According to preferred embodiments, the anionic acrylic polymer may be crosslinked (or branched). Suitable examples of acceptable crosslinking agents include, but are not limited to, methylene bisacrylamide (MBA), ethylene glycol diacrylate, polyethylene glycol dimethacrylate, diacrylamide, cyanomethacrylate, vinyloxyethacrylate or methacrylate, formaldehyde, glyoxal, and compositions of the glycidylether type such as ethyleneglycol diglycidylether, or epoxides.

Particularly preferred acrylic thickeners are disclosed in U.S. Patent Application Publication Nos. 2004/0028637 and 2008/0196174. Particularly preferred acrylic thickeners are sodium acrylate/sodium acryloyldimethyl taurate copolymers.

A particularly preferred commercially available product containing an acrylic thickener is that sold under the INCI name Sodium Acrylate/Sodium Acryloyldimethyl Taurate Copolymer & Hydrogenated Polydecene & Sorbitan Laurate & Trideceth-6 which is marketed by Arch Personal Care Products, South Plainfield, N.J., USA under the tradename ViscUp^{®}EZ. Other commercially available products include SEPPIC's Sepiplus S (hydroxyethyl acrylate sodium acryloyldimethyl taurate copolymer and polyisobutene and PEG-7 trimethyloylpropane coconut ether) and Sepinov EMT 10 (hydroxyethyl acrylate sodium acryloyldimethyl taurate copolymer).

According to preferred embodiments of the present invention, the acrylic thickener is in powder form. Suitable examples of such a thickener include Sepinov EMT 10 discussed above and Sepimax Zen (polyacrylate crosspolymer-6).

According to preferred embodiments of the present invention, the acrylic thickener may comprise an acrylamide monomer. For example, SEPPIC's Simulgel 600 (acrylamide/sodium acryloyldimethyltaurate copolymer/isohexadecane/polysorbate 80) is an acceptable thickener.

The (d) acrylic thickener may have at least one hydrophilic unit and at least one fatty-chain unit, and may be chosen from, for example, those comprising at least one fatty-chain allyl ether unit and at least one hydrophilic unit comprising an ethylenic unsaturated anionic monomeric unit, for example, a vinylcarboxylic acid unit and further, for example, be chosen from units derived from acrylic acids, methacrylic acids, and mixtures thereof, wherein the fatty-chain allyl ether unit corresponds to the monomer of formula (I) below:

CH₂=C(R₁)CH₂OBₙR (I)

in which R₁ is chosen from H and CH₃, B is an ethyleneoxy radical, n is chosen from zero and integers ranging from 1 to 100, R is chosen from hydrocarbon-based radicals chosen from alkyl, arylalkyl, aryl, alkylaryl, and cycloalkyl radicals, containing from 10 to 30 carbon atoms, and further, for example, from 10 to 24 carbon atoms and even further, for example, from 12 to 18 carbon atoms.

In one embodiment, a unit of formula (I) is, for example, a unit in which R₁ can be H, n can be equal to 10, and R can be a stearyl (C₁₈) radical.

The polymers of this type are described and prepared, according to an emulsion polymerization process, in patent EP-0 216 479 B2.

In one embodiment, the (d) acrylic thickener, is selected from, for example, polymers formed from 20% to 60% by weight of acrylic acid and/or of methacrylic acid, from 5% to 60% by weight of lower alkyl (meth)acrylates, from 2% to 50% by weight of fatty-chain allyl ether of formula (I), and from 0% to 1% by weight of a crosslinking agent which is a well-known copolymerizable unsaturated polyethylenic monomer, for example, diallyl phthalate, allyl(meth)acrylate, divinylbenzene, (poly)ethylene glycol dimethacrylate, and methylenebisacrylamide.

Examples of such polymers are crosslinked terpolymers of methacrylic acid, of ethyl acrylate, and of polyethylene glycol (10 EO) stearyl ether (Steareth-10), such as those sold by the company Ciba under the names Salcare SC 80 and Salcare SC 90, which are aqueous 30% emulsions of a crosslinked terpolymer of methacrylic acid, of ethyl acrylate, and of steareth-10 allyl ether (40/50/10).

The (d) acrylic thickener may further be chosen, for example, from those comprising at least one hydrophilic unit of unsaturated olefinic carboxylic acid type, and at least one hydrophobic unit of a type such as a (C₁₀-C₃₀) alkyl ester of an unsaturated carboxylic acid. The hydrophilic unit of unsaturated olefinic carboxylic acid type corresponds to, for example, the monomer of formula (II) below: 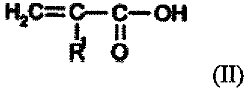 in which R¹ is chosen from H, CH₃, and C₂H₅, i.e., acrylic acid, methacrylic acid, and ethacrylic acid units. The hydrophobic unit of a type such as a (C₁₀-C₃₀) alkyl ester of an unsaturated carboxylic acid corresponds to, for example, the monomer of formula (III) below: 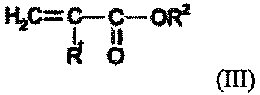 in which is chosen from H, CH₃, and C₂H₅ (i.e., acrylate, methacrylate, and ethacrylate units) and is, for example, chosen from, for example, H (acrylate units) and CH₃ (methacrylate units), and R² is chosen from C₁₀-C₃₀ alkyl radicals, for example, C₁₂-C₂₂ alkyl radicals.

Examples of (C₁₀-C₃₀)alkyl esters of unsaturated carboxylic acids include lauryl acrylate, stearyl acrylate, decyl acrylate, isodecyl acrylate, and dodecyl acrylate, and the corresponding methacrylates, lauryl methacrylate, stearyl methacrylate, decyl methacrylate, isodecyl methacrylate, and dodecyl methacrylate.

According to a preferred embodiment, these thickening polymers are crosslinked.

The polymers of this type are disclosed and prepared, for example, according to U.S. Pat. Nos. 3,915,921 and 4,509,949.

Representative (d) acrylic thickener that can be used may further be chosen from polymers formed from a mixture of monomers comprising:
(i) acrylic acid,
(ii) an ester of formula (III) described above in which R² denotes H or CH₃ and R³ denotes an alkyl radical having from 12 to 22 carbon atoms, and
(iii) a crosslinking agent which is a well-known copolymerizable polyethylenic unsaturated monomer, such as diallyl phthalate, allyl(meth)acrylate, allyl ether of sucrose, allyl ether of pentaerythritol, divinylbenzene, (poly)ethylene glycol dimethacrylate and methylenebisacrylamide.

Use will more particularly be made, among copolymers of this type, of those composed of 95 to 60% by weight of acrylic acid (hydrophilic unit), 4 to 40% by weight of C₁₀-C₃₀ alkyl acrylate (hydrophobic unit) and 0 to 6% by weight of crosslinking polymerizable monomer or else of those composed of 98 to 96% by weight of acrylic acid (hydrophilic unit), 1 to 4% by weight of C₁₀-C₃₀ alkyl acrylate (hydrophobic unit) and 0.1 to 0.6% by weight of crosslinking polymerizable monomer, such as those described above.

Preference is very particularly given according to the present invention, among the said polymers above, to (crosslinked) acrylate/ C₁₀-C₃₀ alkyl acrylate copolymers (INCI name: Acrylates/ C10-30 Alkyl Acrylate Crosspolymer), such as the products sold by Lubrizol under the trade names Pemulen^{®} EZ-4U, Pemulen^{®} TR1, Pemulen^{®} TR2, and Carbopol^{®} Ultrez 20 Polymer.

It is preferable that the (d) acrylic thickener be selected from polyacrylate crosspolymer-6, acrylates/C 10-30 alkyl acrylate crosspolymer and a mixture thereof.

The amount of the (d) acrylic thickener(s) in the composition according to the present invention may be 0.01% by weight or more, preferably 0.05% by weight or more, and more preferably 0.1% by weight or more relative to the total weight of the composition.

On the other hand, the amount of the (d) acrylic thickener(s) in the composition according to the present invention may be 5% by weight or less, preferably 3% by weight or less, and more preferably 1% by weight or less relative to the total weight of the composition.

Thus, the amount of the (d) acrylic thickener(s) in the composition according to the present invention may range from 0.01% to 5% by weight, preferably from 0.05% to 3% by weight, and more preferably from 0.1% to 1% by weight relative to the total weight of the composition.

### [Water]

The composition according to the present invention comprises (e) water.

The (e) water can form an aqueous phase of the composition according to the present invention.

If the composition according to the present invention is in the form of an O/W emulsion, the (e) water in the composition according to the present invention can form a continuous aqueous phases in the O/W emulsion.

The amount of the (e) water in the composition according to the present invention may be 50% by weight or more, preferably 60% by weight or more, and more preferably 70% by weight or more, relative to the total weight of the composition.

On the other hand, the amount of the (e) water in the composition according to the present invention may be 90% by weight or less, preferably 85% by weight or less, and more preferably 80% by weight or less, relative to the total weight of the composition.

The amount of (e) water in the composition according to the present invention may range from 50% to 90% by weight, preferably from 60% to 85% by weight, more preferably from 70% to 80% by weight, relative to the total weight of the composition.

### [Polyol]

The composition according to the present invention may further comprise at least one polyol. A single type of polyol may be used, but two or more different types of polyol may be used in combination.

The term "polyol" here means an alcohol having two or more hydroxy groups, and does not encompass a saccharide or a derivative thereof. The derivative of a saccharide includes a sugar alcohol which is obtained by reducing one or more carbonyl groups of a saccharide, as well as a saccharide or a sugar alcohol in which the hydrogen atom or atoms in one or more hydroxy groups thereof has or have been replaced with at least one substituent such as an alkyl group, a hydroxyalkyl group, an alkoxy group, an acyl group or a carbonyl group.

The polyol may be a C₂-C ₁₂ polyol, preferably a C₂-C₉ polyol, comprising at least 2 hydroxy groups, and preferably 2 to 5 hydroxy groups.

The polyol may be a natural or synthetic polyol. The polyol may have a linear, branched or cyclic molecular structure.

The polyol may be selected from glycerins and derivatives thereof, and glycols and derivatives thereof. The polyol may be selected from the group consisting of glycerin, diglycerin, polyglycerin, ethyleneglycol, diethyleneglycol, propyleneglycol, dipropyleneglycol, butyleneglycol, pentyleneglycol, hexyleneglycol, 1,3-propanediol, 1,5-pentanediol, polyethyleneglycol (5 to 50 ethyleneoxide groups), and sugars such as sorbitol.

The amount of the polyol(s) in the composition according to the present invention may be 0.01% by weight or more, preferably 0.05% by weight or more, and more preferably 0.1 % by weight or more, relative to the total weight of the composition.

On the other hand, the amount of the polyol(s) in the composition according to the present invention may be 20% by weight or less, preferably 15% by weight or less, and more preferably 10% by weight or less, relative to the total weight of the composition.

Thus, the polyol(s) may be present in the composition according to the present invention in an amount ranging from 0.01% to 20% by weight, and preferably from 0.05% to 15% by weight, such as from 0.1% to 10% by weight, relative to the total weight of the composition.

### [Other Ingredients]

The composition according to the present invention may contain one or more monoalcohols which are in the form of a liquid at room temperature (25°C), such as for example linear or branched monoalcohols comprising from 1 to 6 carbon atoms, such as ethanol, propanol, butanol, isopropanol, isobutanol, pentanol, and hexanol.

The amount of the monoalcohol(s) in the composition according to the present invention may be 0.01% by weight or more, preferably 0.1% by weight or more, and more preferably 1% by weight or more, relative to the total weight of the composition.

On the other hand, the amount of the monoalcohol(s) in the composition according to the present invention may be 15% by weight or less, preferably 10% by weight or less, and more preferably 5% by weight or less, relative to the total weight of the composition.

Thus, the amount of the monoalcohol(s) in the composition according to the present invention may range from 0.01% to 15% by weight, preferably from 0.1% to 10% by weight, and more preferably from 1% to 5% by weight, relative to the total weight of the composition.

The composition according to the present invention may also include various adjuvants conventionally used in cosmetic and dermatological compositions, such as anionic, non-ionic, cationic, and amphoteric or zwitterionic polymers, cationic and amphoteric surfactants, antioxidants, coloring agents, chelating agents, sequestering agents, fragrances, dispersing agents, conditioning agents, film-forming agents, preservatives, co-preservatives, and mixtures thereof, except for the ingredients as explained above.

### (Preparation)

The composition according to the present invention can be prepared by mixing the essential ingredient(s) as explained above, and optional ingredient(s), if necessary, as explained above.

The method and means to mix the above essential and optional ingredients are not limited. Any conventional method and means can be used to mix the above essential and optional ingredients to prepare the composition according to the present invention.

### [Form]

The form of the composition according to the present invention is not particularly limited, and may take various forms such as an emulsion (O/W or W/O form), an aqueous gel, an aqueous solution, or the like.

It is preferable that the composition according to the present invention be in the form of an emulsion. It is more preferable that the composition according to the present invention be in the form of an O/W emulsion which comprises fatty phases dispersed in a continuous aqueous phase. The dispersed fatty phases can be oil droplets in the aqueous phase.

The O/W architecture or structure, which consists of fatty phases dispersed in an aqueous phase, has an external aqueous phase, and therefore if the composition according to the present invention has the O/W architecture or structure, it can provide a pleasant feeling during use because of the feeling of immediate freshness that the aqueous phase can provide.

The composition according to the present invention can have a viscosity of from 100 to 2,000 mPa.s at ambient temperature (25°C) under atmospheric pressure (760 mmHg). The viscosity can be measured with a common viscometer such as Brookfield LVDV-III U with Spindle #62, 5 rpm at room temperature (25°C).

### [Process and Use]

It is preferable that the composition according to the present invention be a cosmetic or dermatological composition, preferably a cosmetic composition, and more preferably a cosmetic composition for a keratin substance such as skin.

The composition according to the present invention can be used for a non-therapeutic process, such as a cosmetic process, for treating a keratin substance such as skin, hair, mucous membranes, nails, eyelashes, eyebrows and/or scalp, by being applied to the keratin substance.

Thus, the present invention also relates to a cosmetic process for treating a keratin substance such as skin, comprising the step of applying the composition according to the present invention to the keratin substance.

The present invention may also relate to a use of the composition according to the present invention as a cosmetic product or in a cosmetic product such as care products, washing products, make-up products, make-up-removing products, for body and/or facial skin and/or mucous membranes and/or the scalp and/or the hair and/or the nails and/or the eyelashes and/or the eyebrows.

In other words, the composition according to the present invention can be used, as it is, as a cosmetic product. Alternatively, the composition according to the present invention can be used as an element of a cosmetic product. For example, the composition according to the present invention can be added to or combined with any other elements to form a cosmetic product.

The care product may be a lotion, in particular a milky lotion, a cream, a hair tonic, a hair conditioner, a sun screening agent, and the like. The washing product may be a shampoo, a face wash, a hand wash and the like. The make-up product may be a foundation, a mascara, a lipstick, a lip gloss, a blusher, an eye shadow, a nail varnish, and the like. The make-up-removing product may be a make-up cleansing agent and the like.

Another aspect of the present invention relates to a use of:
(a) at least one oil with a specific gravity at 25°C of more than 0.96 and preferably more than 0.97;
(b) at least one polyglyceryl fatty acid ester having an HLB of 8 to 11
(c) at least one anionic surfactant as above-defined; and
(d) at least one acrylic thickener
   for the manufacture of a stable composition, with a viscosity of from 100 to 2,000 mPa.s and improved texture such as smooth feeling to touch after application, comprising
(e) water.

Another aspect of the present invention also relates to a process for preparing a stable composition with a viscosity of from 100 to 2,000 mPa.s and improved texture such as a smooth feeling to the touch after application, comprising a step of mixing:
(a) at least one oil;
(b) at least one polyglyceryl fatty acid ester having an HLB of 8 to 11;
(c) at least one anionic surfactant as above-defined;
(d) at least one acrylic thickener; and
(e) water,
wherein
the specific gravity of the (a) oil(s) at 25°C is more than 0.96 and preferably more than 0.97.

The above explanations regarding ingredients (a) to (e), as well as the optional ingredients, for the composition according to the present invention can apply to those for the above uses and processes according to the present invention. The explanations regarding the preparation and forms of the composition according to the present invention can also apply to those of the composition described in the above uses and processes.

### EXAMPLES

The present invention will be described in more detail by way of examples which however should not be construed as limiting the scope of the present invention.

### [Examples 1 and 2 and Comparative Examples 1-7]

The following compositions according to Examples 1 and 2 and Comparative Examples 1-7, shown in Table 1, were prepared by mixing the ingredients shown in Table 1 as follows:
(1) mixing the ingredients of Phase A at 70-75 °C to form a uniform mixture of Phase A;
(2) adding the ingredients of Phase B to the uniform mixture of Phase A at 75-80°C followed by mixing with a homogenizer at 70-75°C to form a uniform mixture of Phases A and B;
(3) adding the ingredients of Phase C to the uniform mixture of Phases A and B followed by mixing by a homogenizer at 70-75°C to form a uniform mixture of Phases A, B and C;
(4) cooling the uniform mixture of Phases A, B and C to about 30°C; and
(5) adding the ingredient of Phase D to the uniform mixture of Phases A, B and C at about 30°C followed with a homogenizer.

The compositions according to Examples 1 and 2 and Comparative Examples 1-7 were in the form of an O/W emulsion.

The numerical values for the amounts of the components shown in Table 1 are all based on "% by weight" as active raw materials.

**Table 1**

| | Ingredients | Ex. 1 | Ex. 2 | Comp. Ex. 1 | Comp. Ex. 2 | Comp. Ex. 3 | Comp. Ex. 4 | Comp. Ex. 5 | Comp. Ex. 6 | Comp. Ex. 7 |
|---|---|---|---|---|---|---|---|---|---|---|
| B | PG-6 Dioleate (HLB: 9.8) | 0.9 | 0.9 | - | - | - | 0.9 | 0.9 | 0.9 | 0.9 |
| B | PG-6 Caprylate (HLB: 15) | - | - | 0.9 | - | - | - | - | - | - |
| B | PG-3 Beeswax (HLB: 4-5) | - | - | - | 0.9 | - | - | - | - | - |
| B | Glyceryl Stearate (and) PG-100 Stearate (HLB: 11) | - | - | - | - | 0.9 | - | - | - | - |
| A | Sodium Dilauramidoglutamide Lysine*1 | 0.32 | 0.32 | 0.32 | 0.32 | 0.32 | - | 0.32 | 0.32 | 0.32 |
| B | Caprylic/Capric/Succinic Triglyceride (oil) | 7.5 | 7.5 | 7.5 | 7.5 | 7.5 | 7.5 | 5 | 7.5 | 7.5 |
| B | Diisopropyl Adipate (oil) | 1.25 | 1.25 | 1.25 | 1.25 | 1.25 | 1.25 | - | 1.25 | 1.25 |
| B | Butyrospermum Parkii (shea) Butter (oil) | 1.25 | 1.25 | 1.25 | 1.25 | 1.25 | 1.25 | 5 | 1.25 | 1.25 |
| C | Acrylates/C 10-30 Alkyl Acrylate Crosspolymer | 0.2 | - | 0.2 | 0.2 | 0.2 | 0.2 | 0.2 | - | - |
| C | Polyacrylate Crosspolymer-6 | - | 0.2 | - | - | - | - | - | - | - |
| C | Xanthan Gum | - | - | - | - | - | - | - | - | 0.2 |
| A | Water | qsp 100 | qsp 100 | qsp 100 | qsp 100 | qsp 100 | qsp 100 | qsp 100 | qsp 100 | qsp 100 |
| A | Glycerin | 3 | 3 | 3 | 3 | 3 | 3 | 3 | 3 | 3 |
| A | Butylene Glycol | 5 | 5 | 5 | 5 | 5 | 5 | 5 | 5 | 5 |
| A | Adenosine | 0.1 | 0.1 | 0.1 | 0.1 | 0.1 | 0.1 | 0.1 | 0.1 | 0.1 |
| A | Mannose | 2.5 | 2.5 | 2.5 | 2.5 | 2.5 | 2.5 | 2.5 | 2.5 | 2.5 |
| A | Phenoxyethanol | 0.6 | 0.6 | 0.6 | 0.6 | 0.6 | 0.6 | 0.6 | 0.6 | 0.6 |
| B | Pentaerythrityl Tetra-di-t-butyl hydroxyhydrocinnamate | 0.07 | 0.07 | 0.07 | 0.07 | 0.07 | 0.07 | 0.07 | 0.07 | 0.07 |
| A | Chlorphenesin | 0.2 | 0.2 | 0.2 | 0.2 | 0.2 | 0.2 | 0.2 | 0.2 | 0.2 |
| A | Citric Acid | 0.02 | 0.02 | 0.02 | 0.02 | 0.02 | 0.02 | 0.02 | 0.02 | 0.02 |
| A | Sodium Hydroxide | 0.027 | 0.027 | 0.027 | 0.027 | 0.027 | 0.027 | 0.027 | 0.027 | 0.027 |
| D | Fragrance | 0.04 | 0.04 | 0.04 | 0.04 | 0.04 | 0.04 | 0.04 | 0.04 | 0.04 |
| Average Specific Gravity of Oils (25 °C) | | 0.9875 | 0.9875 | 0.9875 | 0.9875 | 0.9875 | 0.9875 | 0.95 | 0.9875 | 0.9875 |
| Viscosity (measured value) | | 1266 | 356 | 250 | 2100 | 2980 | 3010 | 1300 | 0 | 3839 |
| Viscosity (evaluation) | | Good | Good | Good | Poor | Poor | Poor | Good | Poor | Poor |
| Stability | | Very Good | Very Good | Poor | Poor | Poor | Very Poor | Poor | Very Poor | Poor |
| Texture | | Very Good | Very Good | Poor | Good | Good | Very Good | Very Good | Very Good | Very Good |

| | | | | | | | | | | |
|---|---|---|---|---|---|---|---|---|---|---|
| *1 PELLICER LB-30G (ASAHI KASEI FINE CHEM CO., LTD.): WATER / SODIUM DILAURAMIDOGLUTAMIDE LYSINE / BUTYLENE GLYCOL = 66.7/ 27.3 / 6 (parts by weight) | | | | | | | | | | |

### [Evaluations]

### (Viscosity)

The viscosity of each of the compositions according to Examples 1 and 2 and Comparative Examples 1-7 was measured with a viscometer (Brookfield LVDV-III U with Spindle #62, 5 rpm at room temperature (25°C). The measured viscosity values were classified in the following categories.

| | |
|---|---|
| Good: | from 100 to 2,000 mPa.s |
| Poor: | less than 100 mPa.s or more than 2,000 mPa.s |

The results are shown in Table 1.

### (Stability)

Each of the compositions according to Examples 1 and 2 and Comparative Examples 1-7 was filled into four glass bottles, and each of the glass bottles was held under temperature conditions of 4°C, 25°C, 40°C, and 45°C, respectively, for 2 months.

Each glass bottle was then investigated for the degree of change (in terms of transparency, color, odor, and pH), and evaluated by the following criteria.

| | |
|---|---|
| Very Good: | Almost the same condition as production. |
| Good: | Change in color, odor, and pH could be somewhat observed. No separating or creaming aspect was observed. |
| Poor: | Change in color, odor, and pH could be clearly observed. Separating or creaming aspect could also be clearly observed. |
| Very Poor: | Change in color, odor, and pH could be remarkably noticed. Separating or creaming aspect could be remarkably noticed. |

The results are shown in Table 1.

### (Texture)

Each panelist applied the compositions according to Examples 1 and 2 and Comparative Examples 1-7 onto the skin to evaluate the texture for each composition, and graded them from 1 (poor) to 5 (excellent); they were then classified in the following 4 categories based on the average of the grade.

| | |
|---|---|
| Very Good (5.0 to 4.0): | Very smooth feeling is felt after application. |
| Good (3.9 to 3.0): | Smooth feeling is felt after application. |
| Poor (2.9 to 2.0): | Little smooth feeling is felt after application. |
| Very Poor (1.9 to 1.0): | Sticky feeling is felt after application. |

The results are shown in Table 1.

The compositions according to Examples 1 and 2 which comprise all the ingredients (a) to (e) explained above can achieve a low viscosity such as 100 to 2,000 mPa.s, while they were stable and were able to provide excellent texture such as a smooth feeling to the touch after the application of the compositions.

The composition according to Comparative Example 1 which comprises a polyglyceryl fatty acid ester with an HLB value of more than 11 was not stable and could not provide excellent texture such as a smooth feeling to the touch after the application of the composition.

The composition according to Comparative Example 2 which comprises a polyglyceryl fatty acid ester with an HLB value of less than 8 could not achieve a low viscosity such as 100 to 2,000 mPa.s and was not stable.

The composition according to Comparative Example 3 which comprises a nonionic surfactant which is different from a polyglyceryl fatty acid ester could not achieve a low viscosity such as 100 to 2,000 mPa.s and was not stable, although the nonionic surfactant has an HLB value of 11.

The composition according to Comparative Example 4 which lacks the (c) anionic surfactant could not achieve a low viscosity such as 100 to 2,000 mPa.s, and was not stable.

The composition according to Comparative Example 5 which does not use the (a) oils with an average value of the specific viscosities thereof is more than 0.95 was not stable.

The composition according to Comparative Example 6 which lacks the (d) acrylic thickener had an overly low viscosity (0 mPa.s), and was not stable.

The composition according to Comparative Example 7 which comprises a thickener which is different from the (d) acrylic thickener could not achieve a low viscosity such as 100 to 2,000 mPa.s, and was not stable.

## Claims

1. A composition comprising:
(a) at least one oil;
(b) at least one polyglyceryl fatty acid ester having an HLB of 8 to 11;
(c) at least one anionic surfactant;
(d) at least one acrylic thickener; and
(e) water,
wherein
the specific gravity of the (a) oil(s) at 25°C is more than 0.96 and preferably more than 0.97, and
the (c) anionic surfactant is selected from the compounds represented by the formula (A): 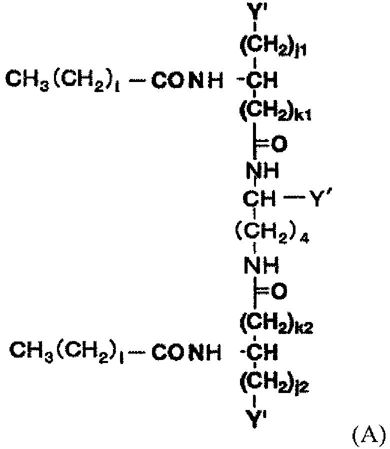 wherein
Y' independently denotes a carboxylic acid group or an alkaline salt of a carboxylic acid group such as a sodium salt of a carboxylic acid group,
j1, k1,j2 and k2 denote integers such that (j1, k1, j2, k2) = any of (2,0,2,0), (2,0,0,2), (0,2,2,0) and (0,2,0,2), and
l denotes an integer from 6 to 16.

2. The composition according to Claim 1, wherein the (a) oil is selected from triglyceride oils.

3. The composition according to Claim 1 or 2, wherein the amount of the (a) oil(s) in the composition ranges from 0.01% to 25% by weight, preferably from 0.1% to 20% by weight, and more preferably from 1% to 15% by weight, relative to the total weight of the composition.

4. The composition according to any one of Claims 1 to 3, wherein the fatty acid moiety of the (b) polyglyceryl fatty acid ester comprises 12 to 24 carbon atoms, preferably 14 to 22 carbon atoms, and more preferably 16 to 20 carbon atoms.

5. The composition according to any one of Claims 1 to 4, wherein the (b) polyglyceryl fatty acid ester comprises 4 to 10 glycerol units, preferably 4 to 9 glycerol units, and more preferably 4 to 8 glycerol units.

6. The composition according to any one of Claims 1 to 5, wherein the amount of the (b) polyglyceryl fatty acid ester(s) in the composition ranges from 0.01% to 10% by weight, preferably from 0.05% to 5% by weight, and more preferably from 0.1% to 2% by weight, relative to the total weight of the composition.

7. The composition according to any one of Claims 1 to 6, wherein the (c) anionic surfactant is selected from the group consisting of sodium dilauramidoglutamide lysine, sodium dimyristoylglutamide lysine, and sodium distearoylglutamide lysine, and a mixture thereof.

8. The composition according to any one of Claims 1 to 7, wherein the amount of the (c) anionic surfactant(s) in the composition ranges from 0.01% to 10% by weight, preferably from 0.03% to 5% by weight, and more preferably from 0.05% to 0.5% by weight, relative to the total weight of the composition.

9. The composition according to any one of Claims 1 to 8, wherein the (d) acrylic thickener is selected from polyacrylate crosspolymer-6, acrylates/C10-30 alkyl acrylate crosspolymer and a mixture thereof.

10. The composition according to any one of Claims 1 to 9, wherein the amount of the (d) acrylic thickener(s) in the composition ranges from 0.01% to 5% by weight, preferably from 0.05% to 3% by weight, and more preferably from 0.1% to 1% by weight, relative to the total weight of the composition.

11. The composition according to any one of Claims 1 to 10, wherein the amount of the (e) water in the composition ranges from 50% to 90% by weight, preferably from 60% to 85% by weight, and more preferably from 70% to 80% by weight, relative to the total weight of the composition.

12. The composition according to any one of Claims 1 to 11, wherein the composition is in the form of an O/W emulsion.

13. The composition according to any one of Claims 1 to 12, wherein the composition is a cosmetic composition, preferably a cosmetic composition for a keratin substance, and more preferably a cosmetic composition for skin.

14. A cosmetic process for treating a keratin substance, comprising the step of applying the composition according to any one of Claims 1 to 13 to the keratin substance.

## Patentansprüche

1. Zusammensetzung, umfassend:
(a) wenigstens ein Öl;
(b) wenigstens einen Polyglycerin-Fettsäureester mit einem HLB-Wert von 8 bis 11;
(c) wenigstens ein anionisches Tensid;
(d) wenigstens ein als Acryl vorliegendes Verdickungsmittel; und
(e) Wasser,
wobei
die relative Dichte des/r (a) Öls/e bei 25 °C mehr als 0,96 und vorzugsweise mehr als 0,97 beträgt und
das (c) anionische Tensid aus den Verbindungen, die durch die Formel (A) dargestellt sind, ausgewählt ist: 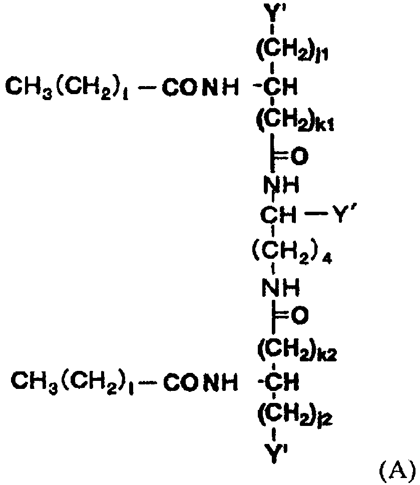
wobei Y' unabhängig eine Carbonsäuregruppe oder ein Alkalisalz einer Carbonsäuregruppe, wie etwa ein Natriumsalz einer Carbonsäuregruppe, angibt,
j1, k1, j2 und k2 ganze Zahlen angeben, sodass (j1, k1, j2, k2) = ein beliebiges von (2,0,2,0), (2,0,0,2), (0,2,2,0) und (0,2,0,2), und
l eine ganze Zahl von 6 bis 16 angibt.

2. Zusammensetzung nach Anspruch 1, wobei das (a) Öl aus Triglyceridölen ausgewählt ist.

3. Zusammensetzung nach Anspruch 1 oder 2, wobei die Menge des/r (a) Öls/e in der Zusammensetzung im Bereich von 0,01 Gew.-% bis 25 Gew.-%, vorzugsweise von 0,1 Gew.-% bis 20 Gew.-% und besonders bevorzugt von 1 Gew.-% bis 15 Gew.-%, bezogen auf das Gesamtgewicht der Zusammensetzung, liegt.

4. Zusammensetzung nach einem der Ansprüche 1 bis 3, wobei die Fettsäurekomponente des (b) Polyglycerin-Fettsäureesters 12 bis 24 Kohlenstoffatome, vorzugsweise 14 bis 22 Kohlenstoffatome und besonders bevorzugt 16 bis 20 Kohlenstoffatome umfasst.

5. Zusammensetzung nach einem der Ansprüche 1 bis 4, wobei der (b) Polyglycerin-Fettsäureester 4 bis 10 Glycerineinheiten, vorzugsweise 4 bis 9 Glycerineinheiten und besonders bevorzugt 4 bis 8 Glycerineinheiten umfasst.

6. Zusammensetzung nach einem der Ansprüche 1 bis 5, wobei die Menge des/r (b) Polyglycerin-Fettsäureester(s) in der Zusammensetzung im Bereich von 0,01 Gew.-% bis 10 Gew.-%, vorzugsweise von 0,05 Gew.-% bis 5 Gew.-% und besonders bevorzugt von 0,1 Gew.-% bis 2 Gew.-%, bezogen auf das Gesamtgewicht der Zusammensetzung, liegt.

7. Zusammensetzung nach einem der Ansprüche 1 bis 6, wobei das (c) anionische Tensid aus der Gruppe, die aus Sodium Dilauramidoglutamide Lysine, Sodium Dimyristoylglutamide Lysine und Sodium Distearoylglutamide Lysine und einem Gemisch davon besteht, ausgewählt ist.

8. Zusammensetzung nach einem der Ansprüche 1 bis 7, wobei die Menge des/r (c) anionischen Tensids/e in der Zusammensetzung im Bereich von 0,01 Gew.-% bis 10 Gew.-%, vorzugsweise von 0,03 Gew.-% bis 5 Gew.-% und besonders bevorzugt von 0,05 Gew.-% bis 0,5 Gew.-%, bezogen auf das Gesamtgewicht der Zusammensetzung, liegt.

9. Zusammensetzung nach einem der Ansprüche 1 bis 8, wobei das (d) als Acryl vorliegende Verdickungsmittel aus Polyacrylate Crosspolymer-6, Acrylates/C10-30 Alkyl Acrylate Crosspolymer und einem Gemisch davon ausgewählt ist.

10. Zusammensetzung nach einem der Ansprüche 1 bis 9, wobei die Menge des/r (d) als Acryl vorliegenden Verdickungsmittel(s) in der Zusammensetzung im Bereich von 0,01 Gew.-% bis 5 Gew.-%, vorzugsweise von 0,05 Gew.-% bis 3 Gew.-% und besonders bevorzugt von 0,1 Gew.-% bis 1 Gew.-%, bezogen auf das Gesamtgewicht der Zusammensetzung, liegt.

11. Zusammensetzung nach einem der Ansprüche 1 bis 10, wobei die Menge des (e) Wassers in der Zusammensetzung im Bereich von 50 Gew.-% bis 90 Gew.-%, vorzugsweise von 60 Gew.-% bis 85 Gew.-% und besonders bevorzugt von 70 Gew.-% bis 80 Gew.-%, bezogen auf das Gesamtgewicht der Zusammensetzung, liegt.

12. Zusammensetzung nach einem der Ansprüche 1 bis 11, wobei die Zusammensetzung in Form einer O/W-Emulsion vorliegt.

13. Zusammensetzung nach einem der Ansprüche 1 bis 12, wobei die Zusammensetzung eine kosmetische Zusammensetzung, vorzugsweise eine kosmetische Zusammensetzung für eine Keratinsubstanz und besonders bevorzugt eine kosmetische Zusammensetzung für Haut ist.

14. Kosmetisches Verfahren zur Behandlung einer Keratinsubstanz, das den Schritt des Auftragens der Zusammensetzung nach einem der Ansprüche 1 bis 13 auf die Keratinsubstanz umfasst.

## Revendications

1. Composition comprenant :
(a) au moins une huile ;
(b) au moins un ester polyglycérique d'acides gras ayant un HLB de 8 à 11 ;
(c) au moins un tensioactif anionique ;
(d) au moins un épaississant acrylique ; et
(d) de l'eau,
dans laquelle
la densité spécifique de la ou des (a) huile(s) à 25 °C est supérieure à 0,96 et de préférence supérieure à 0,97, et
le (c) tensioactif anionique est choisi parmi les composés représentés par la formule (A) : 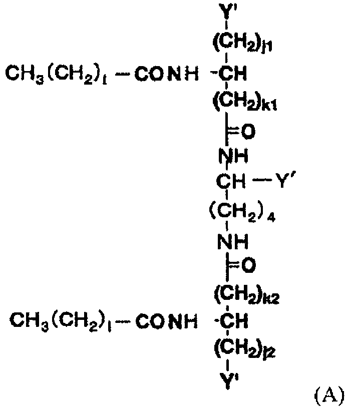 dans laquelle
Y' désigne indépendamment un groupe acide carboxylique ou un sel alcalin d'un groupe acide carboxylique tel qu'un sel de sodium d'un groupe acide carboxylique,
j1, k1, j2 et k2 désignent des entiers tels que (j1, k1, j2, k2) = l'un quelconque de (2, 0, 2, 0), (2, 0, 0, 2), (0, 2, 2, 0) et (0, 2, 0, 2), et
l désigne un nombre entier de 6 à 16.

2. Composition selon la revendication 1, dans laquelle la (a) huile est choisie parmi les huiles triglycérides.

3. Composition selon la revendication 1 ou 2, dans laquelle la quantité de la ou des (a) huile(s) dans la composition va de 0,01 % à 25 % en poids, de préférence de 0,1 % à 20 % en poids, et plus préférentiellement de 1 % à 15 % en poids, par rapport au poids total de la composition.

4. Composition selon l'une quelconque des revendications 1 à 3, dans laquelle la fraction acides gras du (b) ester polyglycérique d'acides gras comprend 12 à 24 atomes de carbone, de préférence 14 à 22 atomes de carbone, et plus préférentiellement 16 à 20 atomes de carbone.

5. Composition selon l'une quelconque des revendications 1 à 4, dans laquelle le (b) ester polyglycérique d'acides gras comprend 4 à 10 motifs glycérol, de préférence 4 à 9 motifs glycérol, et plus préférentiellement 4 à 8 motifs glycérol.

6. Composition selon l'une quelconque des revendications 1 à 5, dans laquelle le quantité du ou des (b) ester(s) polyglycérique(s) d'acides gras dans la composition va de 0,01 % à 10 % en poids, de préférence de 0,05 % à 5 % en poids, et plus préférentiellement de 0,1 % à 2 % en poids, par rapport au poids total de la composition.

7. Composition selon l'une quelconque des revendications 1 à 6, dans laquelle le (c) tensioactif anionique est choisi dans le groupe consistant en dilauramidoglutamide lysine de sodium, dimyristoylglutamide lysine de sodium et distéaroylglutamide lysine de sodium, et un de leurs mélanges.

8. Composition selon l'une quelconque des revendications 1 à 7, dans laquelle la quantité du ou des (c) tensioactif(s) anioniques(s) dans la composition va de 0,01 % à 10 % en poids, de préférence de 0,03 % à 5 % en poids, et plus préférentiellement de 0,05 % à 0,5 % en poids, par rapport au poids total de la composition.

9. Composition selon l'une quelconque des revendications 1 à 8, dans laquelle le (d) épaississant acrylique est choisi parmi le polymère réticulé polyacrylate-6, le polymère réticulé acrylates/acrylate d'alkyle en C₁₀₋₃₀ et un de leurs mélanges.

10. Composition selon l'une quelconque des revendications 1 à 9, dans laquelle la quantité du ou des (c) épaississant(s) acrylique(s) dans la composition va de 0,01 % à 5 % en poids, de préférence de 0,05 % à 3 % en poids, et plus préférentiellement de 0,1 % à 1 % en poids, par rapport au poids total de la composition.

11. Composition selon l'une quelconque des revendications 1 à 10, dans laquelle la quantité de (e) l'eau dans la composition va de 50 % à 90 % en poids, de préférence de 60 % à 85 % en poids, et plus préférentiellement de 70 % à 80 % en poids, par rapport au poids total de la composition.

12. Composition selon l'une quelconque des revendications 1 à 11, dans laquelle la composition se présente sous la forme d'une émulsion E/H.

13. Composition selon l'une quelconque des revendications 1 à 12, dans laquelle la composition est une composition cosmétique, de préférence une composition cosmétique pour une substance kératineuse, et plus préférentiellement une composition cosmétique pour la peau.

14. Procédé cosmétique pour traiter une substance kératineuse, comprenant l'étape d'application de la composition selon l'une quelconque des revendications 1 à 13 sur la substance kératineuse.
